# EUROPEAN PATENT APPLICATION

(11) **EP 4 566 665 A1**
(43) Date of publication of application: **11.06.2025**
(21) Application number: 24829859.8
(22) Date of filing: 22.02.2024
(51) Int. Cl.: A61N 7/00, A61N 7/02

(54) **LARGE-FOCAL-REGION ULTRASOUND THERAPY INSTRUMENT**

(30) Priority: 25.06.2023 CN 202310757822
(71) Applicant: Shenzhen Peninsula Medical Group, Shenzhen, Guangdong 518000 (CN)
(72) Inventor: LI, Yanan, Shenzhen, Guangdong 518000 (CN); PENG, Yujia, Shenzhen, Guangdong 518000 (CN); LEI, Xiaobing, Shenzhen, Guangdong 518000 (CN); DING, Yi, Shenzhen, Guangdong 518000 (CN)
(74) Representative: Osterhoff, Utz
(86) International application number: PCT/CN2024/078149
(87) International publication number: WO 2025/001201

(57) **Abstract**

Disclosed is an ultrasonic treatment device with a large focused area including a housing, a bracket and an ultrasonic transducer. One end of the housing is provided with a treatment window. The bracket is provided in the housing. The ultrasonic transducer is installed at the bracket and is opposite to the treatment window. The ultrasonic transducer is configured to emit to a treatment area to form a focused area. The focused area includes a plurality of focused area units. Each focused area unit is provided with a focused point or a focused line, and a plurality of focused points or focused lines are distributed at intervals.

## Description

### CROSS-REFERENCE TO RELATED APPLICATIONS

The present application claims priority to Chinese Patent Application No. 202310757822.3, filed on June 25, 2023. The disclosures of the above-mentioned applications are incorporated herein by reference in their entireties.

### TECHNICAL FIELD

The present application relates to the technical field of ultrasonic treatment equipment, and in particular to an ultrasonic treatment device with a large focused area.

### BACKGROUND

In recent years, non-invasive beauty technology using a high-intensity focused ultrasound has been widely used. The principle is that the focused ultrasound ceramic wafer installed inside the treatment tip will emit focused ultrasound wave. After being conducted through the liquid conductor, the focused ultrasound wave is focused on the subcutaneous tissue or fat tissue, and forms a "high temperature point" with a very small volume (usually less than 1 mm) and a temperature ranging from 50°C to 75°C on the local subcutaneous tissue or fat tissue, so that the local subcutaneous tissue (skin tissue or fat tissue at a depth ranging from 1 mm to 18 mm) shrinks under the action of high temperature, and the fat tissue will perform "burning fat", thereby achieving the beauty effect of improving skin firmness and losing weight.

The existing ultrasonic treatment device adjusts the frequency of ultrasonic emission on the basis of increasing the treatment area, so that the ultrasonic wave is focused to form a focused area along the vertical direction. However, the focused area in the vertical direction will act on the subcutaneous tissue layer of different depths of the human body, and the treatment effect of the subcutaneous tissue layer at different depths is uneven, resulting in the ultrasonic treatment device with the large focused area being unable to accurately treat the subcutaneous tissue layer at the target depth, so that the ultrasonic treatment device with the large focused area needs more time to effectively act on the subcutaneous tissue layer at the target depth, which greatly affects the treatment efficiency of the ultrasonic treatment device with the large focused area.

### SUMMARY

The main purpose of the present application is to provide an ultrasonic treatment device with the large focused area, aiming to form a focused area in the horizontal direction and accurately treat the subcutaneous tissue layer at a target depth, thereby improving the treatment efficiency of the ultrasonic treatment device with the large focused area.

In view of the above objectives, the present application provides an ultrasonic treatment device with a large focused area, including:
a housing, one end of the housing being provided with a treatment window;
a bracket provided in the housing; and
an ultrasonic transducer. The ultrasonic transducer is installed at the bracket and is opposite to the treatment window; the ultrasonic transducer is configured to emit towards a treatment area to form the large focused area extending in a horizontal direction, the large focused area includes a plurality of focused area units.

Each focused area unit includes a focused point or a focused line distributed at intervals.

In an embodiment, the plurality of focused points or focused lines are distributed at intervals in a rectangular array, a circular array, an elliptical array or an annular array.

In an embodiment, each focused point is provided with a corresponding heat diffusion zone.

In an embodiment, a central temperature of each of the focused points is identical, and the central temperature of each of the focused points is ranged from 48°C to 80°C.

In an embodiment, two adjacent focused points have different center temperatures.

In an embodiment, each of the focused area units is in a rectangular shape, a circular shape, an elliptical shape, or an annular shape.

In an embodiment, shapes of the plurality of focused area units are identical.

In an embodiment, shapes of the plurality of focused area units are different.

In an embodiment, the ultrasonic transducer includes a plurality of transducer elements provided at the bracket; the plurality of transducer elements are distributed at intervals, and each of the transducer elements is configured to form one of the focused points.

In an embodiment, the ultrasonic transducer includes one transducer provided at the bracket, and the transducer is provided with a plurality of emitting surfaces; each of curvature regions corresponding to the plurality of emitting surfaces is different, and each of the curvature regions corresponds to one of the focused points.

In an embodiment, the ultrasonic transducer includes a central transducer and at least one sub-transducer, and the sub-transducer is provided adjacent to the central transducer and is provided outside the central transducer; the central transducer is a spherical transducer, and the sub-transducer is an annular transducer.

In an embodiment, the transducer is a tile-shaped transducer, and the tile-shaped transducer is configured to focus and form a focused line.

In an embodiment, the plurality of the focused points are distributed at equal intervals; and/or an interval between two adjacent focused points is ranged from 0.1 mm to 2 mm.

In an embodiment, the ultrasonic treatment device with the large focused area further includes an acoustic lens provided at the ultrasonic transducer, and the acoustic lens is configured to focus ultrasonic waves emitted by the ultrasonic transducer.

In the technical solution of the present application, the ultrasonic treatment device with a large focused area includes a housing, a bracket and an ultrasonic transducer. One end of the housing is provided with a treatment window. The bracket is provided in the housing. The ultrasonic transducer is installed at the bracket and is opposite to the treatment window. The ultrasonic transducer is configured to emit to a treatment area to form a focused area extending in a horizontal direction. The focused area includes a plurality of focused area units. Each focused area unit is provided with a focused point or a focused line, and a plurality of focused points or focused lines are distributed at intervals. In this way, the energy used in the focused area can act evenly on the subcutaneous tissue layer at the user's target depth, thereby accurately treating the subcutaneous tissue layer at the target depth and improving the treatment efficiency of the ultrasonic treatment device with a large focused area.

### BRIEF DESCRIPTION OF THE DRAWINGS

To more clearly illustrate technical solutions in the embodiments of the present application or the related art, the following will briefly introduce the drawings that need to be used in the description of the embodiments or the related art. Obviously, the drawings in the following description are only some embodiments of the present application. For those skilled in the art, without creative effort, other drawings can be obtained according to the structures shown in these drawings.
FIG. 1 is a schematic structural diagram of an ultrasonic treatment device with a large focused area according to an embodiment of the present application.
FIG. 2 is a schematic structural diagram of an ultrasonic transducer of the ultrasonic treatment device with the large focused area according to a first embodiment of the present application.
FIG. 3 is a schematic structural diagram of the ultrasonic transducer of the ultrasonic treatment device with the large focused area according to a second embodiment of the present application.
FIG. 4 is a schematic structural diagram of an acoustic lens of the ultrasonic treatment device with the large focused area according to an embodiment of the present application.

### Description of reference numbers:

| Reference number | Name | Reference number | Name |
|---|---|---|---|
| 10 | housing | 100 | focused area |
| 20 | bracket | 200 | focused area unit |
| 30 | ultrasonic transducer | 300 | focused point |
| 31 | emitting surface | 400 | focused line |
| 40 | acoustic lens | | |

The realization of the objective, functional characteristics, and advantages of the present application are further described with reference to the accompanying drawings.

### DETAILED DESCRIPTION OF THE EMBODIMENTS

The technical solutions of the embodiments of the present application will be described in detail below with reference to the accompanying drawings. It is obvious that the embodiments described are only some rather than all of the embodiments of the present application. All other embodiments obtained by those skilled in the art based on the embodiments of the present application without creative efforts shall fall within the claimed scope of the present application.

It should be noted that all the directional indications (such as up, down, left, right, front, rear...) in the embodiments of the present application are only used to explain the relative positional relationship, movement, or the like of the components in a certain posture (as shown in the drawings). If the specific posture changes, the directional indication will change accordingly.

Besides, the descriptions associated with, e.g., "first" and "second," in the present application are merely for descriptive purposes, and cannot be understood as indicating or suggesting relative importance or impliedly indicating the number of the indicated technical feature. Therefore, the feature associated with "first" or "second" can expressly or impliedly include at least one such feature. In addition, the technical solutions of the various embodiments can be combined with each other, but the combinations must be based on the realization of those skilled in the art. When the combination of technical solutions is contradictory or cannot be achieved, it should be considered that such a combination of technical solutions does not exist, nor does it fall within the scope of the present application.

The heat diffusion zone formed by the focused ultrasound waves in the subcutaneous tissue layer is generally in an ellipsoidal shape with the focused point as the center. In order to improve the treatment effect of each ultrasound wave and expand the effective area, the existing ultrasonic treatment device with the large focused area that has a single focused point adopts larger single-shot ultrasonic energy to focus the ultrasound wave and form an ultrasound focused area distributed in the vertical direction. The ultrasound focused area distributed in the vertical direction is formed under the subcutaneous tissue layer at the different depths, and the treatment effect on the subcutaneous tissue layer at different depths is also different, resulting in uneven distribution of energy required for treatment. In this way, in order to get better treatment for the subcutaneous tissue layer at the target depth, more ultrasound waves and time are needed for focused treatment, which seriously affects the treatment efficiency of the ultrasonic treatment device with the large focused area.

For this reason, the present application proposes an ultrasonic treatment device with a large focused area.

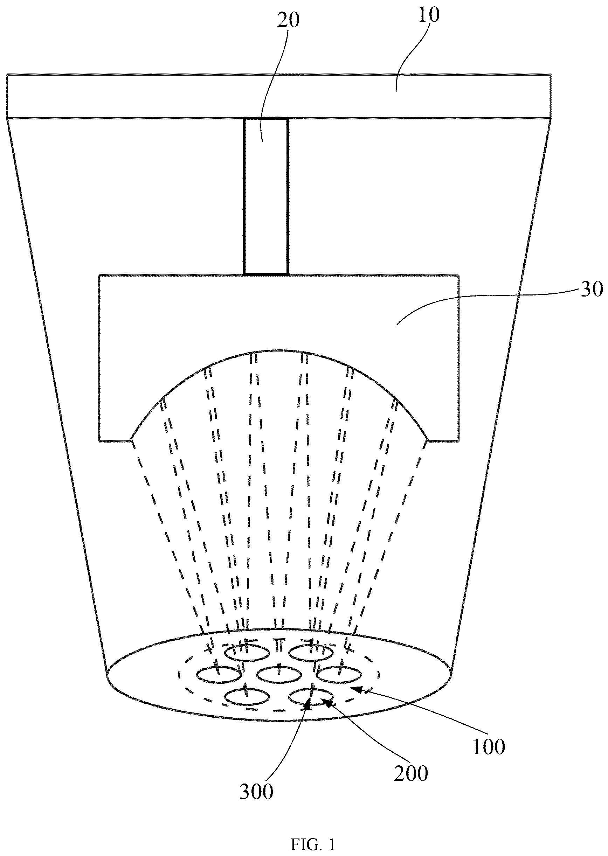

In an embodiment of the present application, as shown in FIG. 1 to FIG. 4, the ultrasonic treatment device with the large focused area includes a housing 10, a bracket 20 and an ultrasonic transducer 30. A treatment window is provided at one end of the housing 10. The bracket 20 is provided in the housing 10. The ultrasonic transducer 30 is installed at the bracket 20 and is opposite to the treatment window. The ultrasonic transducer 30 is configured to emit to the treatment area to form a focused area 100 extending in the horizontal direction. The focused area 100 includes a plurality of focused area units 200. Each focused area unit 200 includes one or more of a focused point 300 or a focused line. The plurality of focused points 300 or focused lines are distributed at intervals.

In an embodiment, the ultrasonic transducer 30 is fixed inside the housing 10 through the bracket 20, and an emission port for emitting ultrasonic waves is provided at the ultrasonic transducer 30 and is opposite to the treatment window of the housing 10. When the ultrasonic transducer 30 is powered on, the ultrasonic transducer 30 will transmit ultrasonic waves to the user's skin (the target plane needing to be treated) through the treatment window of the housing 10, and the ultrasonic waves emitted by the ultrasonic transducer 30 will form a three-dimensional heat diffusion zone in the user's subcutaneous tissue layer. The projection of the three-dimensional heat diffusion zone vertically on the user's skin is a focused area 100 extending in the horizontal direction, and the focused area 100 is formed by a plurality of focused area units 200 disposed and spliced in sequence in the horizontal direction, so that the ultrasonic waves emitted by the ultrasonic transducer 30 can accurately act on the subcutaneous tissue layer at the user's target depth, and will not enter the subcutaneous tissue layer at the non-target depth that does not require treatment.

When each focused point 300 acts on the subcutaneous tissue layer at the target depth, each focused point 300 has a corresponding heat diffusion zone, and the heat diffusion zone has heat that diffuses outwards. The area of the projection of the heat diffusion zone for each focused point 300 perpendicular to the treatment area is the focused area unit 200. Therefore, by distributing a plurality of focused points 300 at intervals, the heat diffusion zones corresponding to two adjacent focused points 300 will not overlap, so that each focused area unit 200 can act on the subcutaneous tissue layer at the target depth respectively. In this way, the energy used by the focused area 100 can act evenly on the subcutaneous tissue layer at the target depth of the user, thereby accurately treating the subcutaneous tissue layer at the target depth, and improving the treatment efficiency of the ultrasonic treatment device with the large focused area.

In an embodiment, as shown in FIG. 1 to FIG. 4, a plurality of focused points 300 or focused lines are disposed in a rectangular array, a circular array, an elliptical array, or an annular array. The focused area 100 formed by the plurality of focused points 300 is also disposed in a rectangular shape, a circular shape, an elliptical shape or an annular shape, and the shape of the focused area 100 is more regular. In this way, not only the operating personnel can control the treatment area more accurately when operating the ultrasonic treatment device with the large focused area, but also the operating personnel can calculate the area of the user's treatment area conveniently, to make accurate data statistics and reference for subsequent treatment plans.

In another embodiment, the plurality of focused points 300 can also be disposed both in a circular array and an annular array. Or, the plurality of focused points 300 can also be disposed in a rectangular array or in an annular array, and the like, and the specific array arrangement combinations are not listed here.

By using a variety of shape arrangement combinations together, the focused area 100 formed by the plurality of focused points 300 can form an irregular shape, which is more conductive for treating the user's irregular treatment area, thereby increasing the treatment area of the ultrasonic treatment device with the large focused area.

In an embodiment, as shown in FIG. 1 to FIG. 4, each focused point 300 is provided with a corresponding heat diffusion area.

In an embodiment, the focused ultrasonic energy will show a thermal effect in the subcutaneous tissue layer of the human body, and heat will diffuse around the focused point 300, that is, the temperature in the center is highest and will gradually decrease in the radial direction. The area where the temperature of the thermal effect can achieve the effective treatment is called the thermal diffusion zone, and the height of the thermal diffusion zone is the height of the space in which the focused point 300 or the focused line plays an effective treatment role in the subcutaneous tissue layer along the vertical direction. By making the height of each thermal diffusion zone equal to the thickness of the focused area unit 200, the single-shot ultrasonic energy corresponding to each focused point 300 can fully act on the subcutaneous tissue layer of the human body, thereby improving the energy utilization rate of the focused area unit 200, improving the treatment effect of the ultrasonic treatment device with the large focused area.

In an embodiment, as shown in FIG. 1 to FIG. 4, the central temperature of all the focused points 300 is identical, and the central temperature of each focused point 300 is in the area from 48°C to 80°C.

In an embodiment, the central temperature of the focused point 300 fall within this area, and the thermal diffusion zone corresponding to the focused point 300 can not only avoid damaging the user's skin, but also achieve an effective treatment effect. The central temperature of the focused point 300 is determined by the specific energy value and the focused distance of a single ultrasonic wave, and the specific energy value and focused distance of a single ultrasonic wave can be calculated by the main control chip of the ultrasonic transducer 30, and the operation is controlled by the main control chip, so that the ultrasonic transducer 30 can accurately treat the user.

In an embodiment, as shown in FIG. 1 to FIG. 4, two adjacent focused points have different center temperatures. In this way, each focused point 300 can be set according to the actual treatment plan of the user, that is, the position with high treatment intensity is provided with a focused point 300 with a higher central temperature for treatment, and the position with low treatment intensity is provided with a focused point 300 with a lower central temperature for treatment, thereby improving the ultrasonic treatment device with the large focused area.

In an embodiment, as shown in FIG. 1 to FIG. 4, each focused area unit 200 is in a rectangular shape, a circular shape, an elliptical shape or an annular shape.

In an embodiment, by setting the emitting surface 31 of different shapes on the transducer or setting the emitting port of different shapes for the emitting tip of the transducer, the focused area unit 200 can be arranged in different shapes. A single ultrasonic wave is emitted by the transducer and forms a three-dimensional heat diffusion area in the subcutaneous tissue layer of the user. The projection of the heat diffusion area of the sphere vertical to the skin surface of the user is a circular focused area unit 200. The projection of the heat diffusion area of the rectangle vertical to the skin surface of the user is a rectangular focused area unit 200. The projection of the heat diffusion area of the ellipse vertical to the skin surface of the user is an ellipsoidal focused area unit 200. The projection of the heat diffusion area of the annulus vertical to the skin surface of the user is an annular focused area unit 200.

The shape of the focused area unit 200 to be a regular shape, such as a rectangular shape, a circular shape, or an ellipsoidal shape, which allows the operating personnel to control the treatment area more conveniently, that is, on the same treatment area, as long as the plurality of focused area units 200 are disposed in sequence in the horizontal direction, there will be no overlapping positions between the two adjacent focused area units 200, thereby improving the treatment efficiency of the ultrasonic treatment device with the large focused area.

In an embodiment, as shown in FIG. 1 to FIG. 4, shapes of the plurality of focused area units 200 are set in the same manner. Each focused area unit 200 is in the same shape, so that not only the plurality of focused area units 200 can form a regular focused area 100, but also the focused area 100 after splicing is more uniform, and the gap between the two adjacent focused area units 200 is smaller, thereby improving the treatment effect of the ultrasonic treatment device with the large focused area.

In another embodiment, as shown in FIG. 1 to FIG. 4, the shapes of the plurality of focused area units 200 are set in different ways. The focused area units 200 of different shapes are combined and disposed according to the actual treatment area and scheme of the current user. In this way, the focused area 100 formed by the focused area units 200 of different shapes can treat the user more specifically, thereby ensuring the effect of ultrasonic treatment.

In the first embodiment, as shown in FIG. 1 to FIG. 4, the ultrasonic transducer 30 includes a plurality of transducer elements provided at the bracket 20, and the plurality of transducer elements are disposed at intervals. Each transducer is configured to form a focused point 300. By fixing a plurality of transducer elements emitting single-shot ultrasonic energy to the bracket 20, each transducer can form a focused point 300. Compared with the previous method of focusing with a single transducer at the same frequency, in this embodiment, a plurality of transducer elements emitting low-frequency ultrasonic energy are used for treatment. The plane focused area 100 formed by the ultrasonic transducer 30 of this structure can provide more uniform energy, which can perform effective and quick treatment without harming the user's skin, thereby improving the treatment effect of the ultrasonic transducer 30.

**In** the second embodiment, as shown in FIG. 1 to FIG. 4, the ultrasonic transducer 30 is a transducer disposed at the bracket 20, and the transducer is provided with a plurality of emitting surfaces 31. The curvature regions corresponding to the plurality of emitting surfaces 31 are different, and each curvature region corresponds to a focused point 300. Specially, in view of the different distances between the plurality of emitting surfaces 31 and the user's skin, a plurality of emitting surfaces 31 with different curvatures are provided at a single transducer, so that the emitting surfaces 31 with different curvatures can form a focused point 300 with equal energy in the user's subcutaneous tissue layer, and then form a plane focused area 100 with uniform energy distribution, thereby improving the treatment effect of the ultrasonic transducer 30.

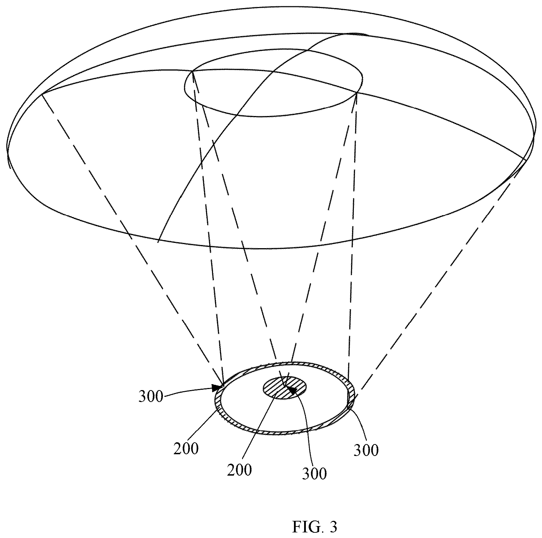

In an embodiment, as shown in FIG. 3, the ultrasonic transducer 30 includes a central transducer and at least one sub-transducer. The sub-transducer is disposed adjacent to the central transducer and is located outside the central transducer. The central transducer is a spherical transducer, and the sub-transducer is an annular transducer.

In an embodiment, more types of combined shapes of focused areas can be formed by combining the central transducer and the sub-transducer. In this way, more types of combined shapes of focused areas are more suitable for complex treatment needs of different users, thereby increasing the treatment area of the ultrasonic treatment device with the large focused area. In this embodiment, the central transducer can be a spherical transducer, and the sub-transducer can be an annular transducer. In this way, the spherical transducer in the center forms a circular focused area on the skin surface, and the annular transducer forms an annular focused area on the skin surface. The circular focused area and the annular focused area are combined. The shapes of the central transducer and the sub-transducer are not limited to the above shapes, but can also be other shapes as long as they meet the actual treatment requirements.

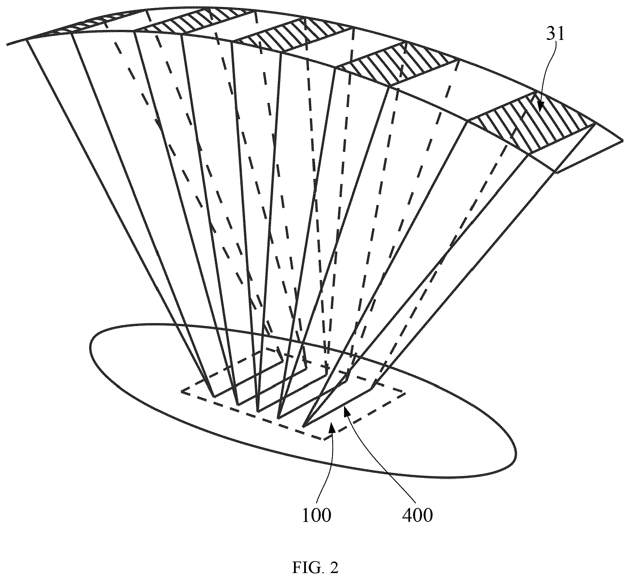

In an embodiment, as shown in FIG. 2, the transducer is a tile-shaped transducer, and the tile-shaped transducer is configured to focus to form a focused line 400. The tile-shaped transducer is focused on the subcutaneous tissue layer of the user, and the projection of the heat diffusion zone focused by the tile-shaped transducer is a line (focused line 400) vertical on the treatment area, and a plurality of focused lines 400 are located on the same plane, thereby converging a focused area 100 shaped in a rectangular plane. The converged plane focused area 100 is easier to be flattened on the treatment area, and the energy of the plane focused area 100 is more uniform, thereby improving the treatment effect of the ultrasonic treatment device with the large focused area.

In an embodiment, as shown in FIG. 1, a plurality of focused points 300 are disposed at equal intervals. The interval between two adjacent focused points 300 ranges from 0.1 mm to 2 mm.

In an embodiment, a plurality of focused points 300 are disposed at the same interval, so that the two focused area units 200 corresponding to the two adjacent focused points 300 will not overlap, which not only makes the energy corresponding to the focused area 100 more uniform, but also facilitates the control on the energy formed by the focused area 100. The specific distance between two adjacent focused points 300 is determined by the position to be treated or the treatment needs of the user. For example, if the position to be treated is the arm, the distance between the two focused points 300 will be smaller, so that energy formed by the focused area 100 is relatively concentrated, and the effect on the skin of the arm with a thicker subcutaneous fat layer is more efficient. If the position to be treated is the face, the distance between the two focused points 300 will be larger, so that energy formed by the focus is relatively dispersed, and the effect on the skin of the face with a thinner subcutaneous fat layer is more delicate, thereby reducing the damage to the skin of the face.

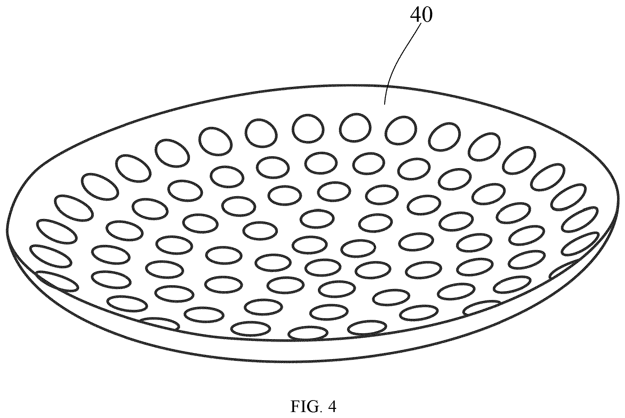

In an embodiment, as shown in FIG. 1 and FIG. 4, the ultrasonic treatment device with the large focused area also includes an acoustic lens 40, which is provided at the ultrasonic transducer 30 and is configured to gather the ultrasonic waves emitted by the ultrasonic transducer 30. Specially, the acoustic lens 40 is a curve-shaped acoustic lens 40, and the acoustic lens 40 is installed at the emitting surface 31 of the transducer. A curve-shaped acoustic lens 40 is installed at the emitting surface 31. After the emitting surface 31 emits ultrasound waves, the ultrasound waves are refracted in the curve-shaped acoustic lens 40 to form a focused area 100 with more uniform and stable energy outside the treatment window, to treat the user, thereby improving the treatment effect of the ultrasonic treatment device with the large focused area.

The above are only some embodiments of the present application, and do not limit the scope of the present application thereto. Under the concept of the present application, any equivalent structural transformation made according to the description and drawings of the present application, or direct/indirect application in other related technical fields shall fall within the claimed scope of the present application.

## Claims

1. An ultrasonic treatment device with a large focused area, comprising:
a housing, wherein one end of the housing is provided with a treatment window;
a bracket provided in the housing; and
an ultrasonic transducer, wherein the ultrasonic transducer is installed at the bracket and is opposite to the treatment window; the ultrasonic transducer is configured to emit towards a treatment area to form the large focused area extending in a horizontal direction, the large focused area comprises a plurality of focused area units;
wherein each focused area unit comprises a focused point or a focused line distributed at intervals.

2. The ultrasonic treatment device with the large focused area according to claim 1, wherein the plurality of focused points or focused lines are distributed at intervals in a rectangular array, a circular array, an elliptical array or an annular array.

3. The ultrasonic treatment device with the large focused area according to claim 2, wherein each focused point is provided with a corresponding heat diffusion zone.

4. The ultrasonic treatment device with the large focused area according to claim 3, wherein a central temperature of each of the focused points is identical, and the central temperature of each of the focused points is ranged from 48°C to 80°C.

5. The ultrasonic treatment device with the large focused area according to claim 3, wherein two adjacent focused points have different center temperatures.

6. The ultrasonic treatment device with the large focused area according to any one of claims 4 to 5, wherein each of the focused area units is in a rectangular shape, a circular shape, an elliptical shape, or an annular shape.

7. The ultrasonic treatment device with the large focused area according to claim 4, wherein shapes of the plurality of focused area units are identical.

8. The ultrasonic treatment device with the large focused area according to claim 5, wherein shapes of the plurality of focused area units are different.

9. The ultrasonic treatment device with the large focused area according to claim 1, wherein the ultrasonic transducer comprises a plurality of transducer elements provided at the bracket; the plurality of transducer elements are distributed at intervals, and each of the transducer elements is configured to form one of the focused points.

10. The ultrasonic treatment device with the large focused area according to claim 1, wherein the ultrasonic transducer comprises one transducer provided at the bracket, and the transducer is provided with a plurality of emitting surfaces; each of curvature regions corresponding to the plurality of emitting surfaces is different, and each of the curvature regions corresponds to one of the focused points.

11. The ultrasonic treatment device with the large focused area according to claim 1, wherein the ultrasonic transducer comprises a central transducer and at least one sub-transducer, and the sub-transducer is provided adjacent to the central transducer and is provided outside the central transducer; the central transducer is a spherical transducer, and the sub-transducer is an annular transducer.

12. The ultrasonic treatment device with the large focused area according to any one of claims 9 to 10, wherein the transducer is a tile-shaped transducer, and the tile-shaped transducer is configured to focus and form a focused line.

13. The ultrasonic treatment device with the large focused area according to claim 1, wherein the plurality of the focused points are distributed at equal intervals; and/or
an interval between two adjacent focused points is ranged from 0.1 mm to 2 mm.

14. The ultrasonic treatment device with the large focused area according to claim 1, further comprising an acoustic lens provided at the ultrasonic transducer, wherein the acoustic lens is configured to focus ultrasonic waves emitted by the ultrasonic transducer.
